# DEMANDE DE BREVET EUROPEEN

(11) **EP 1 484 049 A1**
(43) Date de publication de la demande: **08.12.2004**
(21) Numéro de dépôt: 04291351.7
(22) Date de dépôt: 28.05.2004
(51) Int. Cl.: A61K 7/13, C07D 233/88

(54) **Nouveaux composés imidazoles et utilisation de ces composés pour la teinture de fibres kératiniques**

(30) Priorité: 02.06.2003 FR 0306621
(71) Demandeur: L'OREAL, 75008 Paris (FR)
(72) Inventeur: Mavro, Jacqueline, 94170 Le Perreux (FR); Vidal, Laurent, 75013 Paris (FR); Saunier, Jean-Baptiste, 75014 Paris (FR)
(74) Mandataire: Fevrier, Murielle

(57) **Abrégé**

L'invention a pour objet de nouveaux composés imidazoles utiles comme coupleurs pour la teinture par oxydation des fibres kératiniques.

L'invention a aussi pour objet une composition tinctoriale pour la teinture des fibres kératiniques contenant au moins une base d'oxydation et au moins un coupleur particulier du type imidazole, l'utilisation de cette composition pour la teinture des fibres kératiniques ainsi que le procédé de teinture mettant en oeuvre cette composition.

## Description

L'invention a pour objet de nouveaux composés du type imidazole utiles comme coupleurs pour la teinture par oxydation des fibres kératiniques.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Le but de la présente invention est de fournir de nouveaux coupleurs permettant d'obtenir une gamme encore plus variée de nuances ainsi que des compositions tinctoriales qui présentent des teintures puissantes, uniformes entre la racine et la pointe des cheveux, ayant une bonne chromaticité, peu sélectives et particulièrement résistantes, tout en étant capables d'engendrer des colorations intenses dans des nuances variées, en particulier dans les nuances fondamentales.

Ce but est atteint avec la présente invention qui a pour objet un composé du type imidazole de formule (I) suivante ainsi que ses sels d'addition avec un acide ou une base : 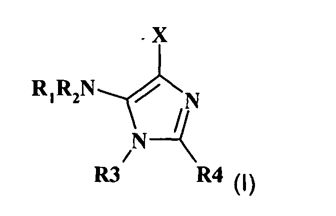 dans laquelle :
- R₁, R₂ et R₄ représentent, identiques ou différents,
   - un atome d'hydrogène ;
   - un radical phényle éventuellement substitué par un ou plusieurs halogènes ou un ou plusieurs radicaux choisis parmi les radicaux hydroxy, alcoxy en C₁-C₄, carboxy, carboxamido, alkyl(C₁-C₄)sulfonyle (-SO₂alkyle), sulfonique (-SO₃H), alkylsulfoxyde (-SO-alkyle), alkylsulfonamido (alkyl(C1-C4)SO₂NH-), N₁₃R₁₄, alkyle en C₁-C₄ éventuellement substitué parmi un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄ ;
   - un radical alkyle en C₁-C₈ linéaire ou ramifié, éventuellement substitué par un ou plusieurs radicaux choisis parmi les radicaux hydroxy, alcoxy en C₁-C₂, carboxy , carboxamido , alkyl(C₁-C₄)sulfonyle, sulfonique, alkylsulfoxyde, alkylsulfonamido ou NR₁₃R₁₄,
- R₄ peut également représenter :
   - un radical NR₂₂R₂₃ où R₂₂ et R₂₃ représentent indépendamment l'un de l'autre un atome d'hydrogène, un radical acyle, un radical alkyle en C₁-C₂ éventuellement substitué par un hydroxy ou un alcoxy en C₁-C₂,
   - un hétérocycle aromatique à 5 ou 6 chaînons éventuellement substitué par un ou plusieurs radicaux choisis parmi les radicaux hydroxy, alcoxy en C₁-C₂, amino, di-méthylamino, 2-hydroxyéthylamino, bis-(2-hydroxyéthyl)amino, chlore,
- R₁ et R₂ peuvent former avec l'atome d'azote avec lequel ils sont attachés un hétérocycle comportant 5 à 8 chaînons dont un ou plusieurs atomes de carbone du cycle carboné peuvent être remplacés par un atome d'oxygène, d'azote, de soufre ou par un groupement SO₂ ; les atomes de carbone dudit cycle peuvent être substitués par un radical R₅ ; le cycle ne comportant pas de liaison peroxyde, ni de radicaux diazo ou nitroso ;
- R₅ représente :
   - un atome d'halogène ;
   - un radical alkyle en C₁-C₄ éventuellement substitué par un ou plusieurs radicaux choisis parmi les radicaux hydroxy, carboxy, alcoxycarbonyle en C₁-C₄, alcoxy en C₁-C₄ ou NR₆R₇ ;
   - un radical carboxy ;
   - un radical carboxamido (alkylCONH-);
   - un radical alkyl(C1-C4)sulfonyle (-SO₂alkyle);
   - un radical alkylsulfonamido (alkyl(C1-C4)SO2NH-)
   - un radical hydroxy ;
   - un radical alcoxy en C₁-C₄ ;
   - un radical hydroxyalcoxy en C₂-C₄ ;
   - un radical aminosulfonyle (NH₂-SO₂-);
   - un radical thioether en C₁-C₄ ;
   - un radical alkyl(C₁-C₄)sulfoxyde (-SOalkyle) ;
   - un radical alkyl(C1-C4)sulfonyle (-SO₂alkyle);
   - un radical NR₈R₉ ;
- R₃ représente
   - un atome d'hydrogène ;
   - un radical alkyl(C₁-C₄)sulfonyle (-SO₂alkyle) ;
   - un radical alkyle en C₁-C₈ linéaire ou ramifié, substitué par un ou plusieurs radicaux choisis parmi les radicaux hydroxy, OR₁₀, carboxy, carboxamido, alkyl(C₁-C₄)sulfonyle, sulfonique (-SO₃H), alkyl(C1-C4)sulfoxyde (-SO-alkyle), alkylsulfonamido (alkyl(C1-C4)SO₂NH-) ou NR₁₁R₁₂ ;
   - un radical phényle ou un hétérocycle aromatique à 5 ou 6 chaînons ces radicaux étant éventuellement substitués par un ou plusieurs halogène, un ou plusieurs radicaux choisis parmi les radicaux hydroxy, alcoxy en C₁-C₄, carboxy, carboxamido, alkyl(C₁-C₄)sulfonyle, sulfonique (-SO₃H) alkylsulfoxyde, thioéther en C₁-C₄, alkylsulfonamido (alkyl(C1-C4)SO₂NH-), NR₁₃R₁₄, alkyle en C₁-C₄ éventuellement substitué par un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄ ;
- R₁₀ représente un radical alkyle en C₁-C₄ éventuellement substitué par un ou plusieurs radicaux choisis parmi les radicaux hydroxy, alcoxy en C₁-C₂, amino, (di)(C₁-C₄)alkylamino ou (poly)hydroxyalkylamino en C₂-C₄ ,
- R₆, R₇, R₈, R₉, R₁₁, R₁₂, R₁₃, R₁₄, représentent, identiques ou différents, un atome d'hydrogène, un radical acyle, un radical carboxamido, un radical alkyle en C₁-C₄ éventuellement substitué par un ou plusieurs radicaux choisis parmi les radicaux hydroxy, alcoxy en C₁-C₄, sulfino, alkyl(C₁-C₄)sulfonyle, alkylsulfonamido, carboxy, carboxamido, alkylsulfoxyde, amino, mono- ou di-(C₁-C₄)alkylamino ou (poly)hydroxyalkylamino en C₂-C₄ ;
- R₁₁ et R₁₂ peuvent également former avec l'atome d'azote qui les porte un cycle de 5 à 8 chaînons éventuellement substitué par un ou plusieurs halogènes, un ou plusieurs radicaux choisis parmi les radicaux hydroxy, alcoxy en C₁-C₄, carboxy, carbamyle, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄,
- X représente un atome d'hydrogène ; un atome d'halogène ; un radical alcoxy en C₁-C₄; un radical phénoxy éventuellement substitué par un ou plusieurs halogènes ou par un ou plusieurs radicaux choisis parmi les radicaux hydroxy, alcoxy en C₁-C₄, carboxy, alcoxycarbonyle en C₁-C₄, carboxamido, sulfino, alkyl(C₁-C₄)sulfonyle, sulfonique, alkylsulfoxyde, thioéther en C₁-C₄, alkylsulfonamido, NR₁₃R₁₄, alkyle en C₁-C₄ ; un thioether en C₁-C₄ éventuellement substitué par un hydroxy, alcoxy en C1-C2, carboxy, sulfonique.

Les composés du type imidazole de formule (I) de l'invention conviennent non seulement pour une utilisation à titre de coupleur pour la teinture d'oxydation des fibres kératiniques, mais en outre qu'ils conduisent à des colorations particulièrement puissantes et peu sélectives. Ils permettent de plus d'obtenir des compositions tinctoriales conduisant à des colorations résistant bien aux diverses agressions que peuvent subir les cheveux, telles que lumière, intempéries, lavage, transpiration. Les compositions de teinture d'oxydation conformes à l'invention permettent de plus d'atteindre des nuances dans une très large palette de couleurs

La présente invention a également pour objet une composition tinctoriale comprenant,
- au moins une base d'oxydation, et
- au moins un coupleur du type imidazole de formule (I) telle que définie ci dessus.

L'invention a aussi pour objet un procédé de teinture mettant en oeuvre cette composition.

Un autre objet de l'invention est l'utilisation des composés de formule (I) pour la teinture par oxydation de fibres kératiniques, en particulier les fibres kératiniques humaines telles que les cheveux.

Dans le cadre de la présente invention, on entend par "chaîne hydrocarbonée ramifiée" une chaîne pouvant former un ou plusieurs cycles carbonés comportant de 3 à 8 chaînons. On entend par chaîne hydrocarbonée insaturée, une chaîne pouvant comporter une ou plusieurs liaisons doubles et/ou une où plusieurs liaisons triples, cette chaîne hydrocarbonée pouvant conduire à des groupements aromatiques.

A titre d'exemple, on peut citer pour les radicaux R₁ et R₂ de la formule(l) un atome d'hydrogène, un radical méthyle, éthyle, (iso)propyle, 2-hydroxyéthyle, 1-hydroxyéthyle, 2-carboxyéthyle, 2-aminoéthyle, 2-(diméthylamino)éthyle, 2-(acylamino)éthyle, 2-méthoxyéthyle, 1,2-dihydroxyéthyle, 1-hydroxy-2-aminoéthyle, 2-hydroxy-1-aminoéthyle, acétyle, NH₂-CO-, diméthylcarboxamido, méthylsulfonyle ou phényle. De préférence, R₁ et R₂ représentent, identiques ou différents, un atome d'hydrogène, les radicaux méthyle, 2-hydroxyéthyle, 2-carboxyéthyle ou 1,2-dihydroxyéthyle.

Selon un mode de réalisation particulier, R₁ et R₂ identiques ou différents, représentent
- un atome d'hydrogène ;
- un radical phényle éventuellement substitué par un ou plusieurs radicaux choisis parmi les radicaux hydroxy, alcoxy en C₁-C₄, carboxamido, alkylsulfoxyde, alkylsulfonamido, N₁₃R₁₄, alkyle en C₁-C₄ éventuellement substitué parmi un ou plusieurs radicaux choisis parmi les radicaux hydroxy, alcoxy en C₁-C₄, carboxamido, alkylsulfonamido ou NR₁₃R₁₄ ;
- un radical alkyle en C₁-C₆ linéaire ou ramifié, éventuellement substitué par un ou plusieurs radicaux choisis parmi les radicaux hydroxy, alcoxy en C₁-C₂, carboxy, carboxamido , alkyl(C₁-C₄)sulfonyle, sulfonique , alkylsulfoxyde , alkylsulfonamido, NR₁₃R₁₄.

Selon un mode de réalisation préférée, R₁ et R₂ représentent, identiques ou différents, un atome d'hydrogène ; un radical phényle ; un radical alkyle en C₁-C₄, linéaire ou ramifié substitué par un ou plusieurs radicaux choisis parmi les radicaux hydroxy, alcoxy en C₁-C₂, amino, di(C₁-C₄)alkylamino, carboxy,un carboxamido, alkylsulfonamido, NR₁₃R₁₄ où R₁₃ et R₁₄ représentent, identiques ou différents, un atome d'hydrogène, acyle, alkyle en C₁-C₂ éventuellement substitué par un hydroxy, un alcoxy en C₁-C₂,

Selon un autre mode de réalisation, R₁ et R₂ forment avec l'atome d'azote avec lequel ils sont attachés un hétérocycle de 5 à 8 chaînons choisis parmi la pyrrolidine, pipéridine, homopipéridine, pipérazine, homopipérazine, lesdits cycles pouvant être substitués par un ou plusieurs radicaux R₅.

A titre d'exemple de ce mode de réalisation particulier, R₁ et R₂ forment ensemble avec l'atome d'azote auquel ils sont rattachés un hétérocycle choisi parmi la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopiperazine, la N-méthyl homopipérazine, la N β-hydroxyéthylhomopipérazine et leurs sels d'addition.

De préférence, R₁ et R₂ forment ensemble avec l'atome d'azote auquel ils sont rattachés un hétérocycle choisi parmi la pyrrolidine, la 3-hydroxypyrrolidine, la 3-aminopyrrolidine, la proline, la 3-hydroxyproline, la pipéridine, la 3 et 4 hydroxypipéridine, l'homopipéridine, l'homopipérazine, la N-méthyl homopipérazine, la N β-hydroxyéthylhomopipérazine et leurs sels d'addition.

A titre d'exemple, on peut citer pour R₃ un atome d'hydrogène, un radical méthyle, éthyle, (iso)propyle, 2-hydroxyéthyle, 1-hydroxyéthyle, 2-carboxyéthyle, 2-aminoéthyle, 2-(diméthylamino)éthyle, 2-(acylamino)éthyle, 2-méthoxyéthyle, 1,2-dihydroxyéthyle, 1-hydroxy-2-aminoéthyle, 2-hydroxy-1-aminoéthyle, méthylsulfonyle ou phényle. De préférence, R₃ représente un atome d'hydrogène, un radical méthyle, 2-hydroxyéthyle, 2-carboxyéthyle, 1,2-dihydroxyéthyle ou phényle.

Selon un mode de réalisation particulier, R₃ est choisi parmi l'atome d'hydrogène, un radical alkyle, un radical hydroxyalkyle, un radical alkylsulfonyle ou un phényle.

A titre d'exemple, on peut citer pour R₄ les radicaux méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle, amino, méthylamino, diméthylamino, 2-hydroxyéthylamino, , bis(2-hydroxyéthyl)amino, un radical phényle, 4-aminophényle, 4-hydroxyphényle, 4-diméthylaminophényle, 4-méthoxyphényle.

Selon un mode de réalisation particulier, R₄ représente un atome d'hydrogène ; un radical amino ; un radical sulfonique; un radical sulfoxyde ; un radical sulfonylamino ; un radical alkyle en C₁-C₆ linéaire ou ramifié éventuellement substitué par un ou plusieurs radicaux choisis parmi un hydroxy, un alcoxy en C₁-C₂, un amino, un carboxy, un carboxamido, un alkylsulfonamido; un radical NR₂₂R₂₃ où R₂₂ et R₂₃ représentent indépendamment l'un de l'autre un atome d'hydrogène, un radical acyle, un radical alkyle en C₁-C₂ éventuellement substitué par un hydroxy ou un alcoxy en C₁-C₂ ; un radical phényle ou un hétérocycle aromatique à 5 ou 6 chaînons éventuellement substitué par un ou plusieurs radicaux choisis parmi les radicaux hydroxy, alcoxy en C₁-C₂, amino, (di-)méthylamino, 2-hydroxyéthylamino, bis-(2-hydroxyéthyl)amino, chlore.

De préférence, R₄ est choisi parmi un atome d'hydrogène, un radical alkyle éventuellement substitué, un radical NR₂₂R₂₃ ou un radical phényle. Selon ce mode de réalisation, R₄ est de préférence choisi parmi un atome d'hydrogène, un radical méthyle, hydroxyméthyle, 2-hydroxyéthyle, 1,2-dihydroxyéthyle, amino, 2-hydroxyéthylamino ou phényle.

Selon un mode de réalisation particulièrement préféré, R₄ est choisi parmi un atome d'hydrogène ou un radical alkyle.

Dans la formule (I) ci dessus, X représente de préférence un atome d'hydrogène, un atome d'halogène tel que le chlore, un radical alcoxy par exemple méthoxy.

Parmi les composés de formule (1), on peut citer à titre d'exemple, les composés suivants :
5-aminoimidazole
5-amino-2-méthylimidazole
5-amino-2-phénylimidazole
5-amino-N-méthylimidazole
5-amino-2-méthyl-N-méthylimidazole
5-amino-2-phényl-N-méthylimidazole
5-amino-N-(2-hydroxyéth-1-yl)imidazole
5-amino-2-méthyl-N-(2-hydroxyéth-1-yl)imidazole
5-amino-2-phényl-N-(2-hydroxyéth-1-yl)imidazole
5-amino-N-(phényl)imidazole
5-amino-2-méthyl-N-(phényl)imidazole
5-amino-2-phényl-N-(phényl)imidazole
5-méthylaminoimidazole
5-méthylamino-2-méthylimidazole
5-méthylamino-2-phénylimidazole
5-méthylamino-N-méthylimidazole
5-méthylamino-2-méthyl-N-méthylimidazole
5-méthylamino-2-phényl-N-méthylimidazole
5-méthylamino-N-(2-hydroxyéth-1-yl)imidazole
5-méthylamino-2-méthyl-N-(2-hydroxyéth-1-yl)imidazole
5-méthylamino-2-phényl-N-(2-hydroxyéth-1-yl)imidazole
5-méthylamino-N-(phényl)imidazole
5-méthylamino-2-méthyl-N-(phényl)imidazole
5-méthylamino-2-phényl-N-(phényl)imidazole
5-diméthylaminoimidazole
5-diméthylamino-2-méthylimidazole
5-diméthylamino-2-phénylimidazole
5-diméthylamino-N-méthylimidazole
5-diméthylamino-2-méthyl-N-méthylimidazole
5-diméthylamino-2-phényl-N-méthylimidazole
5-diméthylamino-N-(2-hydroxyéth-1-yl)imidazole
5-diméthylamino-2-méthyl-N-(2-hydroxyéth-1-yl)imidazole
5-diméthylamino-2-phényl-N-(2-hydroxyéth-1-yl)imidazole
5-diméthylamino-N-(phényl)imidazole
5-diméthylamino-2-méthyl-N-(phényl)imidazole
5-diméthylamino-2-phényl-N-(phényl)imidazole
5-(pyrrolidin-1-yl)imidazole
5-(pyrrolidin-1-yl)-2-méthylimidazole
5-(pyrrolidin-1-yl)-2-phénylimidazole
5-(pyrrolidin-1-yl)-N-méthylimidazole
5-(pyrrolidin-1-yl)-2-méthyl-N-méthylimidazole
5-(pyrrolidin-1-yl)-2-phényl-N-méthylimidazole
5-(pyrrolidin-1-yl)-N-(2-hydroxyéth-1-yl)imidazole
5-(pyrrolidin-1-yl)-2-méthyl-N-(2-hydroxyéth-1-yl)imidazole
5-(pyrrolidin-1-yl)-2-phényl-N-(2-hydroxyéth-1-yl)imidazole
5-(pyrrolidin-1-yl)-N-(phényl)imidazole
5-(pyrrolidin-1-yl)-2-méthyl-N-(phényl)imidazole
5-(pyrrolidin-1-yl)-2-phényl-N-(phényl)imidazole
5-(3-hydroxypyrrolidin-1-yl)imidazole
5-(3-hydroxypyrrolidin-1-yl)-2-méthylimidazole
5-(3-hydroxypyrrolidin-1-yl)-2-phénylimidazole
5-(3-hydroxypyrrolidin-1-yl)-N-méthylimidazole
5-(3-hydroxypyrrolidin-1-yl)-2-méthyl-N-méthylimidazole
5-(3-hydroxypyrrolidin-1-yl )-2-phényl-N-méthylimidazole
5-(3-hydroxypyrrolidin-1-yl)-N-(2-hydroxyéth-1-yl)imidazole
5-(3-hydroxypyrrolidin-1-yl)-2-méthyl-N-(2-hydroxyéth-1-yl)imidazole
5-(3-hydroxypyrrolidin-1-yl)-2-phényl-N-(2-hydroxyéth-1-yl)imidazole
5-(3-hydroxypyrrolidin-1-yl)-N-(phényl)imidazole
5-(3-hydroxypyrrolidin-1-yl)-2-méthyl-N-(phényl)imidazole
5-(3-hydroxypyrrolidin-1-yl)-2-phényl-N-(phényl)imidazole
5-(pipéridin-1-yl)imidazole
5-(pipéridin-1-yl)-2-méthylimidazole
5-(pipéridin-1-yl)-2-phénylimidazole
5-(pipéridin-1-yl)-N-méthylimidazole
5-(pipéridin-1-yl)-2-méthyl-N-méthylimidazole
5-(pipéridin-1-yl)-2-phényl-N-méthylimidazole
5-(pipéridin-1-yl)-N-(2-hydroxyéth-1-yl)imidazole
5-(pipéridin-1-yl)-2-méthyl-N-(2-hydroxyéth-1-yl)imidazole
5-(pipéridin-1-yl)-2-phényl-N-(2-hydroxyéth-1-yl)imidazole
5-(pipéridin-1-yl)-N-(phényl)imidazole
5-(pipéridin-1-yl)-2-méthyl-N-(phényl)imidazole
5-(pipéridin-1-yl)-2-phényl-N-(phényl)imidazole
5-(2-hydroxyéthyl)aminoimidazole
5-(2-hydroxyéthyl)amino-2-méthylimidazole
5-(2-hydroxyéthyl)amino-2-phénylimidazole
5-(2-hydroxyéthyl)amino-N-méthylimidazole
5-(2-hydroxyéthyl)amino-2-méthyl-N-méthylimidazole
5-(2-hydroxyéthyl)amino-2-phényl-N-méthylimidazole
5-(2-hydroxyéthyl)amino-N-(2-hydroxyéth-1-yl)imidazole
5-(2-hydroxyéthyl)amino-2-méthyl-N-(2-hydroxyéth-1-yl)imidazole
5-(2-hydroxyéthyl)amino-2-phényl-N-(2-hydroxyéth-1-yl)imidazole
5-(2-hydroxyéthyl)amino-N-(phényl)imidazole
5-(2-hydroxyéthyl)amino-2-méthyl-N-(phényl)imidazole
5-(2-hydroxyéthyl)amino-2-phényl-N-(phényl)imidazole
5-amino-4-chloroimidazole
5-amino-2-méthyl-4-chloroimidazole
5-amino-2-phényl-4-chloroimidazole
5-amino-N-méthyl-4-chloroimidazole
5-amino-2-méthyl-N-méthyl-4-chloroimidazole
5-amino-2-phényl-N-méthyl-4-chloroimidazole
5-amino-N-(2-hydroxyéth-1-yl)-4-chloroimidazole
5-amino-2-méthyl-N-(2-hyd roxyéth-1-yl )-4-chloroimidazole
5-amino-2-phényl-N-(2-hyd roxyéth-1-yl )-4-chloroimidazole
5-amino-N-(phényl)-4-chloroimidazole
5-amino-2-méthyl-N-(phényl)-4-chloroimidazole
5-amino-2-phényl-N-(phényl)-4-chloroimidazole
5-méthylamino-4-chloroimidazole
5-méthylamino-2-méthyl-4-chloroimidazole
5-méthylamino-2-phényl-4-chloroimidazole
5-méthylamino-N-méthyl-4-chloroimidazole
5-méthylamino-2-méthyl-N-méthyl-4-chloroimidazole
5-méthylamino-2-phényl-N-méthyl-4-chloroimidazole
5-méthylamino-N-(2-hyd roxyéth-1-yl )-4-chloroimidazole
5-méthylamino-2-méthyl-N-(2-hydroxyéth-1 -yl)-4-chloroimidazole
5-méthylamino-2-phényl-N-(2-hydroxyéth-1 -yl)-4-chloroimidazole
5-méthylamino-N-(phényl)-4-chloroimidazole
5-méthylamino-2-méthyl-N-(phényl)-4-chloroimidazole
5-méthylamino-2-phényl-N-(phényl)-4-chloroimidazole
5-diméthylamino-4-chloroimidazole
5-diméthylamino-2-méthyl-4-chloroimidazole
5-diméthylamino-2-phényl-4-chloroimidazole
5-diméthylamino-N-méthyl-4-chloroimidazole
5-diméthylamino-2-méthyl-N-méthyl-4-chloroimidazole
5-diméthylamino-2-phényl-N-méthyl-4-chloroimidazole
5-diméthylamino-N-(2-hydroxyéth-1-yl)-4-chloroimidazole
5-diméthylamino-2-méthyl-N-(2-hydroxyéth-1-yl)-4-chloroimidazole
5-diméthylamino-2-phényl-N-(2-hydroxyéth-1-yl)-4-chloroimidazole
5-diméthylamino-N-(phényl)-4-chloroimidazole
5-diméthylamino-2-méthyl-N-(phényl)-4-chloroimidazole
5-diméthylamino-2-phényl-N-(phényl)-4-chloroimidazole
5-(pyrrolidin-1-yl)-4-chloroimidazole
5-(pyrrolidin-1-yl)-2-méthyl-4-chloroimidazole
5-(pyrrolidin-1-yl)-2-phényl-4-chloroimidazole
5-(pyrrolidin-1-yl)-N-méthyl-4-chloroimidazole
5-(pyrrolidin-1-yl)-2-méthyl-N-méthyl-4-chloroimidazole
5-(pyrrolidin-1-yl)-2-phényl-N-méthyl-4-chloroimidazole
5-(pyrrolidin-1-yl)-N-(2-hydroxyéth-1-yl)-4-chloroimidazole
5-(pyrrolidin-1-yl )-2-méthyl-N-(2-hydroxyéth-1-yl)-4-chloroimidazole
5-(pyrrolidin-1-yl)-2-phényl-N-(2-hydroxyéth-1-yl)-4-chloroimidazole
5-(pyrrolidin-1-yl)-N-(phényl)-4-chloroimidazole
5-(pyrrolidin-1-yl)-2-méthyl-N-(phényl)-4-chloroimidazole
5-(pyrrolidin-1-yl)-2-phényl-N-(phényl)-4-chloroimidazole
5-(3-hydroxypyrrolidin-1-yl)-4-chloroimidazole
5-(3-hydroxypyrrolidin-1-yl)-2-méthyl-4-chloroimidazole
5-(3-hydroxypyrrolidin-1-yl)-2-phényl-4-chloroimidazole
5-(3-hydroxypyrrolidin-1-yl)-N-méthyl-4-chloroimidazole
5-(3-hydroxypyrrolidin-1-yl)-2-méthyl-N-méthyl-4-chloroimidazole
5-(3-hyd roxypyrrolid in-1-yl )-2-phényl-N-méthyl-4-chloroimidazole
5-(3-hydroxypyrrolidin-1-yl)-N-(2-hydroxyéth-1-yl)-4-chloroimidazole
5-(3-hydroxypyrrolidin-1-yl)-2-méthyl-N-(2-hydroxyéth-1-yl)-4-chloroimidazole
5-(3-hydroxypyrrolidin-1-yl)-2-phényl-N-(2-hydroxyéth-1-yl)-4-chloroimidazole
5-(3-hydroxypyrrolidin-1-yl)-N-(phényl)-4-chloroimidazole
5-(3-hydroxypyrrolidin-1-yl)-2-méthyl-N-(phényl)-4-chloroimidazole
5-(3-hydroxypyrrolidin-1-yl)-2-phényl-N-(phényl)-4-chloroimidazole
5-(pipéridin-1-yl)-4-chloroimidazole
5-(pipéridin-1-yl)-2-méthyl-4-chloroimidazole
5-(pipéridin-1-yl)-2-phényl-4-chloroimidazole
5-(pipéridin-1-yl)-N-méthyl-4-chloroimidazole
5-(pipéridin-1-yl)-2-méthyl-N-méthyl-4-chloroimidazole
5-(pipéridin-1-yl)-2-phényl-N-méthyl-4-chloroimidazole
5-(pipéridin-1-yl)-N-(2-hydroxyéth-1-yl)-4-chloroimidazole
5-(pipéridin-1-yl)-2-méthyl-N-(2-hydroxyéth-1-yl)-4-chloroimidazole
5-(pipéridin-1-yl)-2-phényl-N-(2-hydroxyéth-1-yl)-4-chloroimidazole
5-(pipéridin-1-yl)-N-(phényl)-4-chloroimidazole
5-(pipéridin-1-yl)-2-méthyl-N-(phényl)-4-chloroimidazole
5-(pipéridin-1-yl)-2-phényl-N-(phényl)-4-chloroimidazole
5-(2-hydroxyéthyl)amino-4-chloroimidazole
5-(2-hydroxyéthyl)amino-2-méthyl-4-chloroimidazole
5-(2-hydroxyéthyl)amino-2-phényl-4-chloroimidazole
5-(2-hydroxyéthyl)amino-N-méthyl-4-chloroimidazole
5-(2-hydroxyéthyl)amino-2-méthyl-N-méthyl-4-chloroimidazole
5-(2-hydroxyéthyl)amino-2-phényl-N-méthyl-4-chloroimidazole
5-(2-hydroxyéthyl)amino-N-(2-hydroxyéth-1-yl)-4-chloroimidazole
5-(2-hydroxyéthyl)amino-2-méthyl-N-(2-hydroxyéth-1-yl)-4-chloroimidazole
5-(2-hyd roxyéthyl )amino-2-phényl-N-(2-hydroxyéth-1-yl)-4-chloroim idazole
5-(2-hydroxyéthyl)amino-N-(phényl)-4-chloroimidazole
5-(2-hydroxyéthyl)amino-2-méthyl-N-(phényl)-4-chloroimidazole
5-(2-hydroxyéthyl)amino-2-phényl-N-(phényl)-4-chloroimidazole
ainsi que leurs sels d'addition avec un acide ou une base.

Parmi ces composés, les composés suivants sont particulièrement préférés 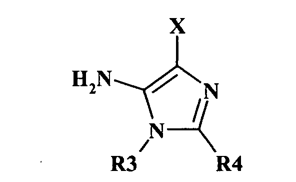 dans laquelle les radicaux X, R3 et R4 sont tels que définis précédemment.

Parmi ces composés, sont particulièrement préférés les composés dans lesquels X est un hydrogène ou un radical alkyle, R3 est un atome d'hydrogène, un radical alkyle, éventuellement substitué par un hydroxy, R4 est un atome d'hydrogène ou un radical alkyle.

A titre d'exemple, ces composés de formule (I) plus particulièrement préférés sont choisis parmi :
5-amino-2-méthylimidazole ;
5-amino-2-méthyl-(2,3-dihydroxypropyl)imidazole ;
5-amino-1,2-diméthylimidazole ;
5-amino-1-(2-hydroxy)éthyl-2-méthylimidazole ;
Ester 2-(5-amino-2-méthyl-imidazol-1-yl)-éthylique de l'acide benzoïque ;
2-(5-amino-2-méthyl-imidazol-4-ylsulfanyl)-acétamide ainsi que leurs sels d'addition avec un acide ou une base.

Les composés de la présente invention sont obtenus par exemple à partir de réactifs imidazole substitués par un radical nitro qui sont réduit par des méthodes classiques de réduction..

La composition de la présente invention est particulièrement utile pour la coloration par oxydation de fibres kératiniques, en particulier de fibres kératiniques humaines.

La composition de teinture par oxydation de la présente invention comprend une ou plusieurs bases d'oxydation classiquement utilisées en teinture d'oxydation. A titre d'exemple, ces bases d'oxydation sont choisies parmi les bases d'oxydation, par exemple les paraphénylènediamines, les bis-phénylalkylènediamines, les para-aminophénols, les ortho-aminophénols, les bases hétérocycliques et leur sels d'addition avec un acide ou une base.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthytoxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène et leurs sels d'addition avec un acide ou une base.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou une base sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide ou une base.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou une base.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide ou une base.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide ou une base.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide ou une base.

La ou les bases d'oxydation sont en général chacune présentes en quantité comprise entre 0,001 à 10 % en poids environ du poids total de la composition tinctoriale, de préférence entre 0,005 et 6 %.

La composition selon l'invention peut contenir un ou plusieurs coupleurs additionnels conventionnellement utilisés pour la teinture de fibres kératiniques. Parmi ces coupleurs additionnels, on peut notamment citer les métaphénylènediamines, les méta-aminophénols, les métadiphénols, les coupleurs naphtaléniques, les coupleurs hétérocycliques autres que ceux du type imidazole selon l'invention et leur sels d'addition avec un acide ou une base.

A titre d'exemple, on peut citer le 2-méthyl 5-aminophénol, le 5-N-(ß-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène et leurs sels d'addition avec un acide ou une base.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 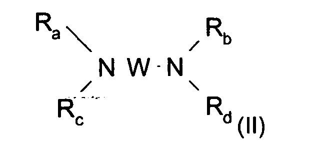 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour obtenir la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les composés de formule (I) peuvent être obtenus selon les méthodes de synthèse connues dans le domaine de la synthèse d'hétérocycle.

A titre d'exemple, les dérivés d'imidazoles alkylés en position 1 sont obtenus selon la méthode décrite dans *Heterocyclic Chemistry, A.R.Katritsky, Chap. Single ring with two heteroatom* par réaction des nitroimidazoles 1 avec le réactif R₃X₁ où R₃ représente un radical alkyle selon la définition ci-dessus et X₁ un groupe nucléofuge tel qu'un halogénure, un O-sulfonate dans un solvant apolaire ou polaire et en présence d'une base telle qu'un alcoolate ou un hydrure métallique à une température comprise entre -20°C et 60°C. Lorsque R3 représente un radical phényle selon la définition ci-dessus et X1 un halogénure, alors un catalyseur à base de palladium (0) ou (II) est ajouté au milieu réactionnel pour réaliser le couplage N-C azote carbone selon la procédure d'Hartwig - Buchwald (Hartwig, J.F. *Synlett* ***1997,*** 329 ;. *Acc. Chem. Res.* ***1998,*** *31,* 805 ; *J. Org. Chem.* ***1996,*** *61,* 7240).

Après bromation par action de brome ou de N-bromosuccinimide ou chloration par action de N-chlorosuccinimide en position 5, les dérivés 3 sont obtenus. L'addition - élimination en position 5 du dérivé imidazolique 3 par un nucléophile de formule X⁻ tel qu'un phénate ou un alcoolate ou encore une thiolate éventuellement substitué tel que défini ci-dessus conduit aux composés nitrés 4.

Ceux-ci sont ensuite soumis à une réaction d'hydrogénation par catalyse hétérogène telle que Pd/C, Pd(II)/C, Ni/Ra, etc... ou encore à une réaction de réduction par un métal tel que par du zinc, fer, étain,etc...( voir Advanced Organic Chemistry, 3^{ème} édition, J. March, et Réduction in organic Chemistry, M ; Hudlicky). La fonctionalisation de l'amine primaire en position 4 en amines secondaires et tertiaires NR₁R₂ ( éventuellement formant un cycle) est réalisée selon les méthodes classiques de synthèse organique ( halogénure d'alkyle, O-sulfonate d'alkyle , trialkylammonium d'alkyle, amination réductrice, etc...voir par exemple *Advanced Organic Chemistry, 3*^{*ème*} *édition, J. March.*

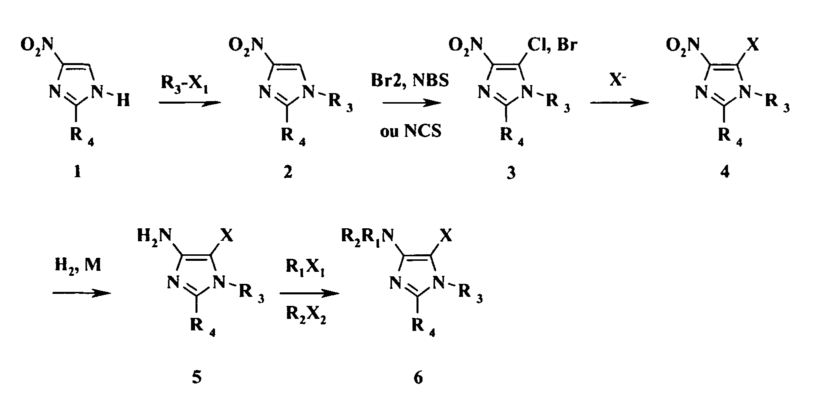

Les exemples qui suivent servent à illustrer l'invention sans toutefois présenter un caractère limitatif.

### EXEMPLES

### Mode opératoire général utilisé :

A une solution hydroalcolique contenant un acide protique tel que l'acide chlorhydrique, le chlorure d'ammonium, l'acide acétique ou l'acide sulfurique et 0,5 g de zinc sous forme de poudre sont ajoutés le dérivé nitroimidazole à une température allant de 20 °C à 80°C. Après consommation totale du produit de départ nitré, le mélange réactionnel est concentré jusqu'à précipitation d'un solide qui est filtré, lavé par de l'éther diisopropylique et séché jusqu'à poids constant.

### Exemple 1.

Le composé ci dessous a été obtenu par réduction dans les conditions décrites ci dessus d'un composé 5-nitro 2-méthylimidazole
5-amino-2-méthylimidazole :

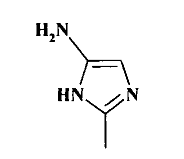

### Exemple 2.

Le composé ci dessous a été obtenu par réduction dans les conditions décrites ci dessus d'un composé 5-nitro 2-méthyl-(2,3-dihydroxypropyl)imidazole
5-amino-2-méthyl-(2,3-dihydroxypropyl)imidazole

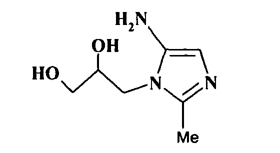

### Exemple 3.

Le composé ci dessous a été obtenu par réduction dans les conditions décrites ci dessus d'un composé 5-nitro 1,2-diméthylimidazole
5-amino-1,2-diméthylimidazole

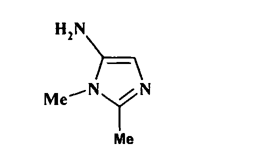

### Exemple 4.

Le composé ci dessous a été obtenu par réduction dans les conditions décrites ci dessus d'un composé 5-nitro 1-(2-hydroxy)éthyl-2-méthylimidazole
5-amino-1-(2-hydroxy)éthyl-2-méthylimidazole

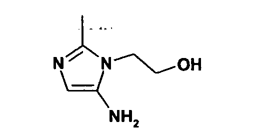

### Exemple 5.

Le composé ci dessous a été obtenu par réduction dans les conditions décrites ci dessus d'un composé Ester 2-(5-nitro-2-méthyl-imidazol-1-yl)-éthylique de l'acide benzoïque

Ester 2-(5-amino-2-méthyl-imidazol-1-yl)-éthylique de l'acide benzoïque

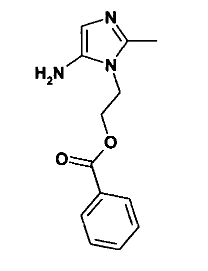

### Exemple 6.

Le composé ci dessous a été obtenu par réduction dans les conditions décrites ci dessus d'un composé 2-(5-nitro-2-méthyl-imidazol-4-ylsulfanyl)-acétamide
2-(5-amino-2-méthyl-imidazol-4-ylsulfanyl)-acétamide

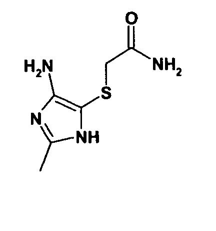

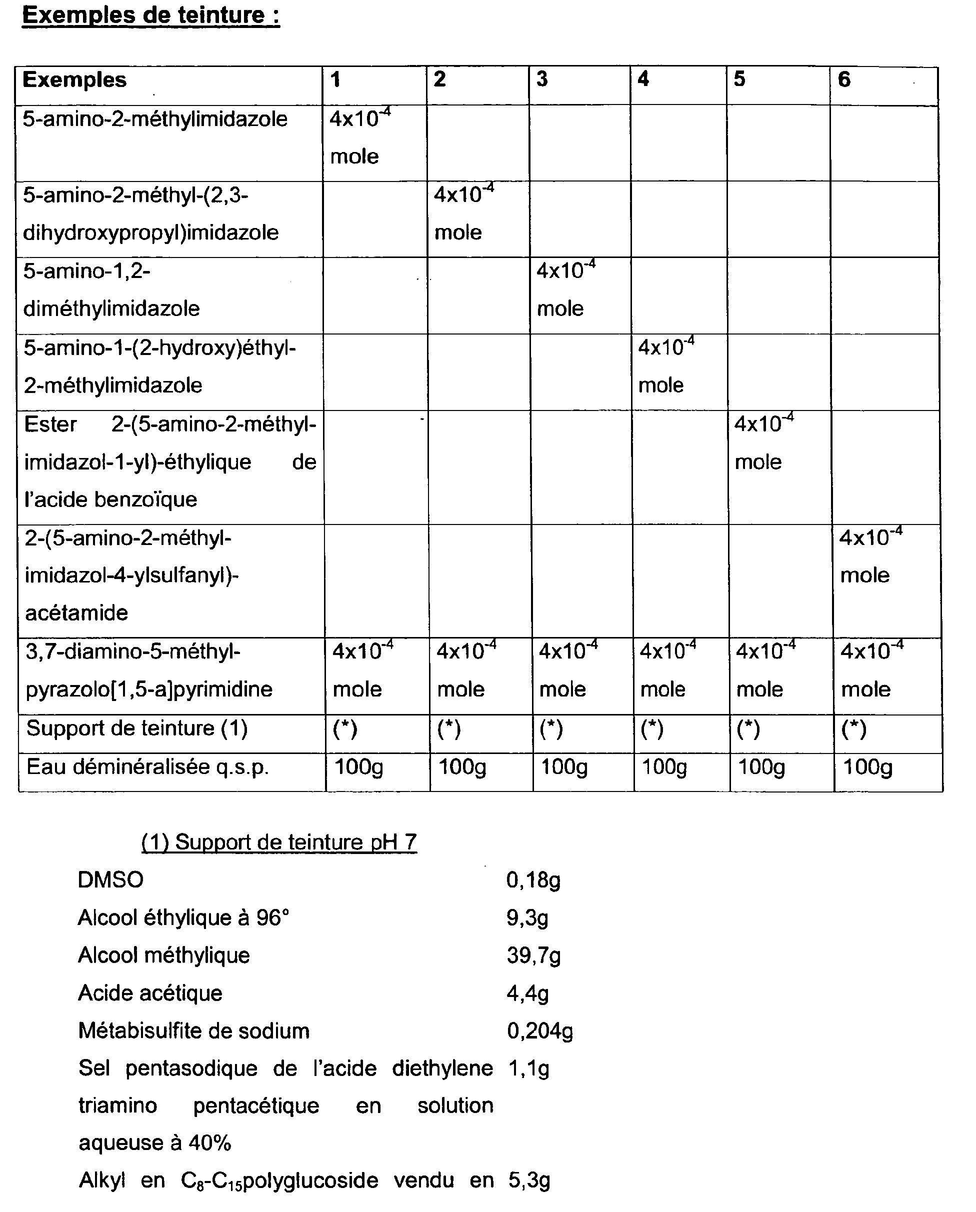

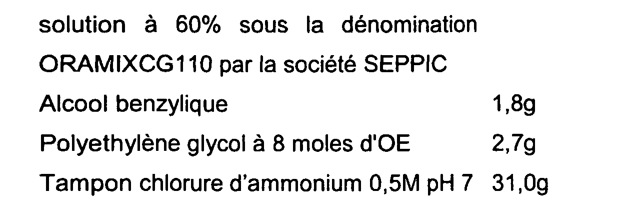

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

| Exemple | 1 | 2 | 3 | 4 | 5 | 6 |
|---|---|---|---|---|---|---|
| Nuance observée | Brun rosé | Rose orangé | Rose orangé | Rose orangé | Rose orangé | Brun rosé |

## Revendications

1. Composé du type imidazole de formule (I) suivante ainsi que ses sels d'addition avec un acide ou une base : 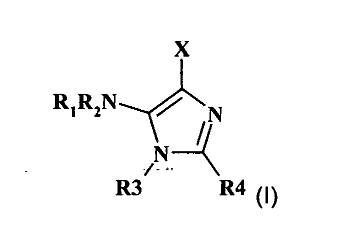 dans laquelle :
• R₁, R₂ et R₄ représentent, identiques ou différents,
- un atome d'hydrogène ;
- un radical phényle éventuellement substitué par un ou plusieurs halogènes ou un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido, N₁₃R₁₄, alkyle en C₁-C₄ éventuellement substitué parmi un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄ ;
- un radical alkyle en C₁-C₈ linéaire ou ramifié, éventuellement substitué par un ou plusieurs radicaux choisis parmi les radicaux hydroxy, alcoxy en C₁-C₂, carboxy, carboxamido , alkyl(C₁-C₄)sulfonyle, sulfonique, alkylsulfoxyde, alkylsulfonamido ou NR₁₃R₁₄,
• R₄ peut également représenter :
- un radical NR₂₂R₂₃ où R₂₂ et R₂₃ représentent indépendamment l'un de l'autre un atome d'hydrogène, un radical acyle, un radical alkyle en C₁-C₂ éventuellement substitué par un hydroxy ou un alcoxy en C₁-C₂,
- un hétérocycle aromatique à 5 ou 6 chaînons éventuellement substitué par un ou plusieurs radicaux choisis parmi les radicaux hydroxy, alcoxy en C₁-C₂, amino, di-méthylamino, 2-hydroxyéthylamino, bis-(2-hydroxyéthyl)amino, chlore,
• R₁ et R₂ peuvent former avec l'atome d'azote avec lequel ils sont attachés un hétérocycle comportant 5 à 8 chaînons dont un ou plusieurs atomes de carbone du cycle carboné peuvent être remplacés par un atome d'oxygène, d'azote, de soufre ou par un groupement SO₂ ; les atomes de carbone dudit cycle peuvent être substitués par un radical R₅ ; le cycle ne comportant pas de liaison peroxyde, ni de radicaux diazo ou nitroso ;
• R₅ représente :
- un atome d'halogène ;
- un radical alkyle en C₁-C₄ éventuellement substitué par un ou plusieurs radicaux choisis parmi les radicaux hydroxy, carboxy, alcoxycarbonyle en C₁-C₄, alcoxy en C₁-C₄ ou NR₆R₇ ;
- un radical carboxy ;
- un radical carboxamido ;
- un radical alkyl(C1-C4)sulfonyle;
- un radical alkylsulfonamido ,
- un radical hydroxy ;
- un radical alcoxy en C₁-C₄ ;
- un radical hydroxyalcoxy en C₂-C₄ ;
- un radical aminosulfonyle ;
- un radical thioether en C₁-C₄ ;
- un radical alkyl(C₁-C₄)sulfoxyde ;
- un radical alkyl(C1-C4)sulfonyle ;
- un radical NR₈R₉ ;
• R₃ représente
- un atome d'hydrogène ;
- un radical alkyl(C₁-C₄)sulfonyle ;
- un radical alkyle en C₁-C₈ linéaire ou ramifié, substitué par un ou plusieurs radicaux choisis parmi les radicaux hydroxy, OR₁₀, carboxy, carboxamido, alkyl(C₁-C₄)sulfonyle, sulfonique, alkyl(C1-C4)sulfoxyde, alkylsulfonamido ou NR₁₁R₁₂ ;
- un radical phényle ou un hétérocycle aromatique à 5 ou 6 chaînons ces radicaux étant éventuellement substitués par un ou plusieurs halogène, un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄ ;
• R₁₀ représente un radical alkyle en C₁-C₄ éventuellement substitué par un ou plusieurs radicaux choisis parmi les radicaux hydroxy, alcoxy en C₁-C₂, amino, (di)(C₁-C₄)alkylamino ou (poly)hydroxyalkylamino en C₂-C₄ ,
• R₆, R₇, R₈, R₉, R₁₁, R₁₂, R₁₃, R₁₄, représentent, identiques ou différents, un atome d'hydrogène, un radical acyle, un radical carboxamido, un radical alkyle en C₁-C₄ éventuellement substitué par un ou plusieurs radicaux choisis parmi les radicaux hydroxy, alcoxy en C₁-C₄, sulfino, alkyl(C₁-C₄)sulfonyle, alkylsulfonamido, carboxy, carboxamido, alkylsulfoxyde, amino, mono- ou di-(C₁-C₄)alkylamino ou (poly)hydroxyalkylamino en C₂-C₄ ;
• R₁₁ et R₁₂ peuvent également former avec l'atome d'azote qui les porte un cycle de 5 à 8 chaînons éventuellement substitué par un ou plusieurs halogènes, un ou plusieurs radicaux choisis parmi les radicaux hydroxy, alcoxy en C₁-C₄, carboxy, carbamyle, alkyl(C₁-C₄)sulfonyle, sulfonique, alkylsulfoxyde, thioéther en C₁-C₄, alkylsulfonamido, NR₁₃R₁₄, alkyle en C₁-C₄ éventuellement substitué par un ou plusieurs radicaux choisis parmi les radicaux hydroxy, alcoxy en C₁-C₄, carboxy, carboxamido, alkyl(C₁-C₄)sulfonyle, sulfonique, alkylsulfoxyde, alkylsulfonamido ou NR₁₃R₁₄,
• X représente un atome d'hydrogène ; un atome d'halogène ; un radical alcoxy en C₁-C₄; un radical phénoxy éventuellement substitué par un ou plusieurs halogènes ou par un ou plusieurs radicaux choisis parmi les radicaux hydroxy, alcoxy en C₁-C₄, carboxy, alcoxycarbonyle en C₁-C₄, carboxamido, sulfino, alkyl(C₁-C₄)sulfonyle, sulfonique, alkylsulfoxyde, thioéther en C₁-C₄, alkylsulfonamido, NR₁₃R₁₄, alkyle en C₁-C₄ ; un thioether en C₁-C₄ éventuellement substitué par un hydroxy, alcoxy en C1-C2, carboxy, sulfonique.

2. Composé selon la revendication 1 dans lequel R₁ et R₂, identiques ou différents, représentent
- un atome d'hydrogène ;
- un radical phényle éventuellement substitué par un ou plusieurs radicaux choisis parmi les radicaux hydroxy, alcoxy en C₁-C₄, carboxamido, alkylsulfoxyde, alkylsulfonamido, N₁₃R₁₄, alkyle en C₁-C₄ éventuellement substitué parmi un ou plusieurs radicaux choisis parmi les radicaux hydroxy, alcoxy en C₁-C₄, carboxamido, alkylsulfonamido ou NR₁₃R₁₄ ;
- un radical alkyle en C₁-C₆linéaire ou ramifié, éventuellement substitué par un ou plusieurs radicaux choisis parmi les radicaux hydroxy, alcoxy en C₁-C₂, carboxy, carboxamido , alkyl(C₁-C₄)sulfonyle, sulfonique , alkylsulfoxyde , alkylsulfonamido, NR₁₃R₁₄.

3. Composé selon la revendication 2 dans lequel R₁ et R₂, identiques ou différents, représentent un atome d'hydrogène ; un radical phényle ; un radical alkyle en C₁-C₄, linéaire ou ramifié substitué par un ou plusieurs radicaux choisis parmi les radicaux hydroxy, alcoxy en C₁-C₂, amino, di(C₁-C₄)alkylamino, carboxy,un carboxamido, alkylsulfonamido, NR₁₃R₁₄ où R₁₃ et R₁₄ représentent, identiques ou différents, un atome d'hydrogène, acyle, un alkyle en C₁-C₂ éventuellement substitué par un hydroxy, un alcoxy en C₁-C₂.

4. Composé selon la revendication 3 dans lequel R₁ et R₂, identiques ou différents, représentent un atome d'hydrogène ; un radical phényle ; un radical alkyle en C₁-C₄, linéaire ou ramifié substitué par un ou plusieurs radicaux choisis parmi les radicaux hydroxy, alcoxy en C₁-C₂, amino, di(C₁-C₄)alkylamino, carboxy, carboxamido, alkylsulfonamido, NR₁₃R₁₄ où R₁₃ et R₁₄ représentent, identiques ou différents, un atome d'hydrogène, acyle, un alkyle en C₁-C₂ éventuellement substitué par un hydroxy, un alcoxy en C₁-C₂.

5. Composé selon la revendication 4 dans lequel R₁ et R₂ représentent, identiques ou différents, un atome d'hydrogène, les radicaux méthyle, 2-hydroxyéthyle, 2-carboxyéthyle ou 1,2-dihydroxyéthyle.

6. Composé selon la revendication 1 dans lequel R₁ et R₂ forment avec l'atome d'azote avec lequel ils sont attachés un hétérocycle de 5 à 8 chaînons choisis parmi la pyrrolidine, pipéridine, homopipéridine, pipérazine, homopipérazine, lesdits cycles pouvant être substitués par un ou plusieurs radicaux R₅.

7. Composé selon la revendication 6 dans lequel R₁ et R₂ forment ensemble avec l'atome d'azote auquel ils sont rattachés un hétérocycle choisi parmi la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino-pyrrolidine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopiperazine, la N-méthyl homopipérazine, la N β-hydroxyéthylhomopipérazine et leurs sels d'addition.

8. Composé selon la revendication 6 ou 7 dans lequel R₁ et R₂ forment ensemble avec l'atome d'azote auquel ils sont rattachés un hétérocycle choisi parmi la pyrrolidine, la 3-hydroxypyrrolidine, la 3-aminopyrrolidine, la proline, la 3-hydroxyproline, la pipéridine, la 3 et 4 hydroxypipéridine, l'homopipéridine, l'homopipérazine, la N-méthyl homopipérazine, la N β-hydroxyéthylhomopipérazine et leurs sels d'addition.

9. Composé selon l'une quelconque des revendications 1 à 8 dans lequel R₃ est choisi parmi l'atome d'hydrogène, un radical alkyle, un radical hydroxyalkyle, un radical alkylsulfonyle ou un phényle.

10. Composé selon l'une quelconque des revendications 1 à 9 dans lequel R₄ représente un atome d'hydrogène ; un radical amino ; un radical sulfonique; un radical sulfoxyde ; un radical sulfonylamino ; un radical alkyle en C₁-C₆ linéaire ou ramifié éventuellement substitué par un ou plusieurs radicaux choisis parmi un hydroxy, un alcoxy en C₁-C₂, un amino, un carboxy, un carboxamido, un alkylsulfonamido; un radical NR₂₂R₂₃ où R₂₂ et R₂₃ représentent indépendamment l'un de l'autre un atome d'hydrogène, un radical acyle, un radical alkyle en C₁-C₂ éventuellement substitué par un hydroxy ou un alcoxy en C₁-C₂ ; un radical phényle ou un hétérocycle aromatique à 5 ou 6 chaînons éventuellement substitué par un ou plusieurs radicaux choisis parmi les radicaux hydroxy, alcoxy en C₁-C₂, amino, (di-)méthylamino, 2-hydroxyéthylamino, bis-(2-hydroxyéthyl)amino, chlore.

11. Composé selon la revendication 10 dans lequel R₄ est choisi parmi un atome d'hydrogène ou un radical alkyle.

12. Composé selon l'une quelconque des revendications 1 à 11, dans lequel X représente un atome d'hydrogène, un atome d'halogène, un radical alcoxy.

13. Composé selon l'une quelconque des revendications 1 à 12, choisi parmi les composés suivants :
5-aminoimidazole
5-amino-2-méthylimidazole
5-amino-2-phénylimidazole
5-amino-N-méthylimidazole
5-amino-2-méthyl-N-méthylimidazole
5-amino-2-phényl-N-méthylimidazole
5-amino-N-(2-hydroxyéth-1-yl)imidazole
5-amino-2-méthyl-N-(2-hydroxyéth-1 -yl)imidazole
5-amino-2-phényl-N-(2-hydroxyéth-1 -yl)imidazole
5-amino-N-(phényl)imidazole
5-amino-2-méthyl-N-(phényl)imidazole
5-amino-2-phényl-N-(phényl)imidazole
5-méthylaminoimidazole
5-méthylamino-2-méthylimidazole
5-méthylamino-2-phénylimidazole
5-méthylamino-N-méthylimidazole
5-méthylamino-2-méthyl-N-méthylimidazole
5-méthylamino-2-phényl-N-méthylimidazole
5-méthylamino-N-(2-hydroxyéth-1-yl)imidazole
5-méthylamino-2-méthyl-N-(2-hydroxyéth-1-yl)imidazole
5-méthylam ino-2-phényl-N-(2-hydroxyéth-1-yl)imidazole
5-méthylamino-N-(phényl)imidazole
5-méthylamino-2-méthyl-N-(phényl)imidazole
5-méthylamino-2-phényl-N-(phényl)imidazole
5-diméthylaminoimidazole
5-diméthylamino-2-méthylimidazole
5-diméthylamino-2-phénylimidazole
5-diméthylamino-N-méthylimidazole
5-diméthylamino-2-méthyl-N-méthylimidazole
5-diméthylamino-2-phényl-N-méthylimidazole
5-diméthylamino-N-(2-hydroxyéth-1-yl)imidazole
5-diméthylamino-2-méthyl-N-(2-hydroxyéth-1 -yl)imidazole
5-diméthylamino-2-phényl-N-(2-hydroxyéth-1 -yl)imidazole
5-diméthylamino-N-(phényl)imidazole
5-diméthylamino-2-méthyl-N-(phényl)imidazole
5-diméthylamino-2-phényl-N-(phényl)imidazole
5-(pyrrolidin-1-yl)imidazole
5-(pyrrolidin-1-yl)-2-méthylimidazole
5-(pyrrolidin-1-yl)-2-phénylimidazole
5-(pyrrolidin-1-yl)-N-méthylimidazole
5-(pyrrolidin-1-yl)-2-méthyl-N-méthylimidazole
5-(pyrrolidin-1-yl)-2-phényl-N-méthylimidazole
5-(pyrrolidin-1-yl)-N-(2-hydroxyéth-1-yl)imidazole
5-(pyrrolidin-1-yl)-2-méthyl-N-(2-hydroxyéth-1-yl)imidazole
5-(pyrrolidin-1-yl)-2-phényl-N-(2-hydroxyéth-1-yl)imidazole
5-(pyrrolidin-1-yl)-N-(phényl)imidazole
5-(pyrrolidin-1-yl)-2-méthyl-N-(phényl)imidazole
5-(pyrrolidin-1-yl)-2-phényl-N-(phényl)imidazole
5-(3-hydroxypyrrolidin-1-yl)imidazole
5-(3-hydroxypyrrolidin-1-yl)-2-méthylimidazole
5-(3-hydroxypyrrolidin-1-yl)-2-phénylimidazole
5-(3-hydroxypyrrolidin-1-yl )-N-méthylimidazole
5-(3-hydroxypyrrolidin-1-yl )-2-méthyl-N-méthylimidazole
5-(3-hydroxypyrrolidin-1-yl )-2-phényl-N-méthylimidazole
5-(3-hydroxypyrrolidin-1-yl)-N-(2-hydroxyéth-1-yl)imidazote
5-(3-hydroxypyrrolidin-1-yl)-2-méthyl-N-(2-hydroxyéth-1-yl)imidazole
5-(3-hydroxypyrrolidin-1-yl)-2-phényl-N-(2-hydroxyéth-1-yl)imidazole
5-(3-hydroxypyrrolidin-1-yl)-N-(phényl)imidazole
5-(3-hydroxypyrrolidin-1-yl)-2-méthyl-N-(phényl)imidazole
5-(3-hydroxypyrrolidin-1 -yl)-2-phényl-N-(phényl)imidazole
5-(pipéridin-1-yl)imidazole
5-(pipéridin-1-yl)-2-méthylimidazole
5-(pipéridin-1-yl)-2-phénylimidazole
5-(pipéridin-1-yl)-N-méthylimidazole
5-(pipéridin-1-yl)-2-méthyl-N-méthylimidazole
5-(pipéridin-1-yl)-2-phényl-N-méthylimidazole
5-(pipéridin-1-yl)-N-(2-hydroxyéth-1-yl)imidazole
5-(pipéridin-1-yl)-2-méthyl-N-(2-hydroxyéth-1-yl)imidazole
5-(pipéridin-1-yl )-2-phényl-N-(2-hydroxyéth-1-yl)imidazole
5-(pipéridin-1-yl)-N-(phényl)imidazole
5-(pipéridin-1-yl)-2-méthyl-N-(phényl)imidazole
5-(pipéridin-1-yl)-2-phényl-N-(phényl)imidazole
5-(2-hydroxyéthyl)aminoimidazole
5-(2-hydroxyéthyl)amino-2-méthylimidazole
5-(2-hydroxyéthyl)amino-2-phénylimidazole
5-(2-hydroxyéthyl)amino-N-méthylimidazole
5-(2-hydroxyéthyl)amino-2-méthyl-N-méthylimidazole
5-(2-hydroxyéthyl)amino-2-phényl-N-méthylimidazole
5-(2-hydroxyéthyl)amino-N-(2-hydroxyéth-1-yl)imidazole
5-(2-hydroxyéthyl)amino-2-méthyl-N-(2-hydroxyéth-1-yl)imidazole
5-(2-hydroxyéthyl)amino-2-phényl-N-(2-hydroxyéth-1-yl)imidazole
5-(2-hydroxyéthyl)amino-N-(phényl)imidazole
5-(2-hydroxyéthyl)amino-2-méthyl-N-(phényl)imidazole
5-(2-hydroxyéthyl)amino-2-phényl-N-(phényl)imidazole
5-amino-4-chloroimidazole
5-amino-2-méthyl-4-chloroimidazole
5-amino-2-phényl-4-chloroimidazole
5-amino-N-méthyl-4-chloroimidazole
5-amino-2-méthyl-N-méthyl-4-chloroimidazole
5-amino-2-phényl-N-méthyl-4-chloroimidazole
5-amino-N-(2-hydroxyéth-1-yl)-4-chloroimidazole
5-amino-2-méthyl-N-(2-hydroxyéth-1-yl)-4-chloroimidazole
5-amino-2-phényl-N-(2-hydroxyéth-1-yl)-4-chloroimidazole
5-amino-N-(phényl)-4-chloroimidazole
5-amino-2-méthyl-N-(phényl)-4-chloroimidazole
5-amino-2-phényl-N-(phényl)-4-chloroimidazole
5-méthylamino-4-chloroimidazole
5-méthylamino-2-méthyl-4-chloroimidazole
5-méthylamino-2-phényl-4-chloroimidazole
5-méthylamino-N-méthyl-4-chloroimidazole
5-méthylamino-2-méthyl-N-méthyl-4-chloroimidazole
5-méthylamino-2-phényl-N-méthyl-4-chloroimidazole
5-méthylamino-N-(2-hydroxyéth-1-yl)-4-chloroimidazole
5-méthylamino-2-méthyl-N-(2-hydroxyéth-1-yl)-4-chloroimidazole
5-méthylamino-2-phényl-N-(2-hydroxyéth-1-yl)-4-chloroimidazole
5-méthylamino-N-(phényl)-4-chloroimidazole
5-méthylamino-2-méthyl-N-(phényl)-4-chloroimidazole
5-méthylamino-2-phényl-N-(phényl)-4-chloroimidazole
5-diméthylamino-4-chloroimidazole
5-diméthylamino-2-méthyl-4-chloroimidazole
5-diméthylamino-2-phényl-4-chloroimidazole
5-diméthylamino-N-méthyl-4-chloroimidazole
5-diméthylamino-2-méthyl-N-méthyl-4-chloroimidazole
5-diméthylamino-2-phényl-N-méthyl-4-chloroimidazole
5-diméthylamino-N-(2-hydroxyéth-1-yl)-4-chloroimidazole
5-diméthylamino-2-méthyl-N-(2-hydroxyéth-1-yl)-4-chloroimidazole
5-diméthylamino-2-phényl-N-(2-hydroxyéth-1-yl)-4-chloroimidazole
5-diméthylamino-N-(phényl)-4-chloroimidazole
5-diméthylamino-2-méthyl-N-(phényl)-4-chloroimidazole
5-diméthylamino-2-phényl-N-(phényl)-4-chloroimidazole
5-(pyrrolidin-1-yl)-4-chloroimidazole
5-(pyrrolidin-1-yl)-2-méthyl-4-chloroimidazole
5-(pyrrolidin-1-yl)-2-phényl-4-chloroimidazole
5-(pyrrolidin-1-yl)-N-méthyl-4-chloroimidazole
5-(pyrrolidin-1-yl)-2-méthyl-N-méthyl-4-chloroimidazole
5-(pyrrolidin-1-yl)-2-phényl-N-méthyl-4-chloroimidazole
5-(pyrrolidin-1-yl)-N-(2-hydroxyéth-1-yl)-4-chloroimidazole
5-(pyrrolid in-1-yl )-2-méthyl-N-(2-hydroxyéth-1-yl )-4-chloroimidazole
5-(pyrrolidin-1-yl)-2-phényl-N-(2-hydroxyéth-1-yl)-4-chloroimidazole
5-(pyrrolidin-1-yl)-N-(phényl)-4-chloroimidazole
5-(pyrrolidin-1-yl)-2-méthyl-N-(phényl)-4-chloroimidazole
5-(pyrrolidin-1-yl)-2-phényl-N-(phényl)-4-chloroimidazole
5-(3-hydroxypyrrolidin-1-yl)-4-chloroimidazole
5-(3-hydroxypyrrolidin-1-yl)-2-méthyl-4-chloroimidazole
5-(3-hydroxypyrrolidin-1-yl)-2-phényl-4-chloroimidazole
5-(3-hydroxypyrrolidin-1-yl)-N-méthyl-4-chloroimidazole
5-(3-hydroxypyrrolidin-1 -yl)-2-méthyl-N-méthyl-4-chloroimidazole
5-(3-hydroxypyrrolidin-1-yl)-2-phényl-N-méthyl-4-chloroimidazole
5-(3-hydroxypyrrolidin-1-yl)-N-(2-hydroxyéth-1-yl)-4-chloroimidazole
5-(3-hydroxypyrrolidin-1-yl)-2-méthyl-N-(2-hydroxyéth-1-yl)-4-chloroimidazole
5-(3-hydroxypyrrolidin-1-yl)-2-phényl-N-(2-hydroxyéth-1-yl)-4-chloroimidazole
5-(3-hydroxypyrrolidin-1-yl)-N-(phényl)-4-chloroimidazole
5-(3-hyd roxypyrrolid in-1-yl)-2-méthyl-N-(phényl )-4-chloroimidazole
5-(3-hydroxypyrrolidin-1-yl)-2-phényl-N-(phényl)-4-chloroimidazole
5-(pipéridin-1-yl)-4-chloroimidazole
5-(pipéridin-1-yl)-2-méthyl-4-chloroimidazole
5-(pipéridin-1-yl)-2-phényl-4-chloroimidazole
5-(pipéridin-1-yl)-N-méthyl-4-chloroimidazole
5-(pipéridin-1-yl)-2-méthyl-N-méthyl-4-chloroimidazole
5-(pipéridin-1-yl)-2-phényl-N-méthyl-4-chloroimidazole
5-(pipéridin-1-yl)-N-(2-hydroxyéth-1-yl)-4-chloroimidazole
5-(pipéridin-1-yl)-2-méthyl-N-(2-hydroxyéth-1-yl)-4-chloroimidazole
5-(pipéridin-1-yl)-2-phényl-N-(2-hydroxyéth-1-yl)-4-chloroimidazole
5-(pipéridin-1-yl)-N-(phényl)-4-chloroimidazole
5-(pipéridin-1-yl)-2-méthyl-N-(phényl)-4-chloroimidazole
5-(pipéridin-1-yl)-2-phényl-N-(phényl)-4-chloroimidazole
5-(2-hydroxyéthyl)amino-4-chloroimidazole
5-(2-hydroxyéthyl)amino-2-méthyl-4-chloroimidazole
5-(2-hydroxyéthyl)amino-2-phényl-4-chloroimidazole
5-(2-hydroxyéthyl)amino-N-méthyl-4-chloroimidazole
5-(2-hydroxyéthyl)amino-2-méthyl-N-méthyl-4-chloroimidazole
5-(2-hydroxyéthyl)amino-2-phényl-N-méthyl-4-chloroimidazole
5-(2-hydroxyéthyl)amino-N-(2-hydroxyéth-1-yl)-4-chloroimidazole
5-(2-hydroxyéthyl )am ino-2-méthyl-N-(2-hydroxyéth-1-yl )-4-chloroim idazole
5-(2-hydroxyéthyl)amino-2-phényl-N-(2-hydroxyéth-1-yl)-4-chloroimidazole
5-(2-hydroxyéthyl)amino-N-(phényl)-4-chloroimidazole
5-(2-hydroxyéthyl)amino-2-méthyl-N-(phényl)-4-chloroimidazole
5-(2-hydroxyéthyl)amino-2-phényl-N-(phényl)-4-chloroimidazole
ainsi que leurs sels d'addition avec un acide ou une base.

14. Composé selon l'une quelconque des revendications 1 à 13 choisi parmi les composés suivants :
5-amino-2-méthylimidazole ;
5-amino-2-méthyl-(2,3-dihydroxypropyl)imidazole ;
5-amino-1,2-diméthylimidazole ;
5-amino-1-(2-hydroxy)éthyl-2-méthylimidazole ;
Ester 2-(5-amino-2-méthyl-imidazol-1-yl)-éthylique de l'acide benzoïque ;
2-(5-amino-2-méthyl-imidazol-4-ylsulfanyl)-acétamide ainsi que leurs sels d'addition avec un acide ou une base.

15. Composé selon l'une quelconque des revendications 1 à 14 de formule 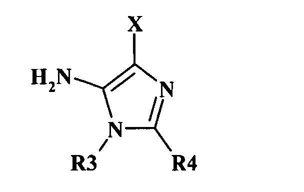 dans laquelle X, R3 et R4 sont tels que définis selon l'une quelconque des revendications 1 à 14.

16. Composé selon la revendication 15 dans laquelle X est un hydrogène ou un radical alkyle, R3 est un atome d'hydrogène, un radical alkyle, éventuellement substitué par un hydroxy, R4 est un atome d'hydrogène ou un radical alkyle.

17. Composition de teinture comprenant, dans un milieu de teinture approprié :
- au moins une base d'oxydation, et
- au moins un coupleur du type imidazole de formule (I) ou l'un de ses sels d'addition correspondants tels que définis dans l'une quelconque des revendications 1 à 16.

18. Composition selon la revendication 17 dans laquelle la ou les bases d'oxydation sont choisies parmi les paraphénylènediamines, les bis-phénylalkylènediamines, les para-aminophénols, les ortho-aminophénols, les bases hétérocycliques et leurs sels d'addition.

19. Composition selon l'une quelconque des revendications 17 ou 18, dans laquelle la ou les bases d'oxydation sont chacune présentes en quantité comprise 0,001 et 10 % en poids du poids total de la composition tinctoriale.

20. Composition selon l'une quelconque des revendications 17 à 19, comprenant un ou plusieurs coupleurs additionnels choisis parmi les métaphénylènediamines, les méta-aminophénols, les métadiphénols, les coupleurs naphtaléniques et les coupleurs hétérocycliques autres que ceux de formule (I) et leurs sels d'addition.

21. Composition selon l'une quelconque des revendications 17 à 20, dans laquelle le ou les coupleurs sont présents chacun en quantité comprise entre 0,001 et 10 % en poids du poids total de la composition tinctoriale.

22. Composition selon l'une quelconque des revendications 17 à 21 comprenant un milieu cosmétiquement acceptable approprié à la teinture des fibres kératiniques.

23. Procédé de teinture d'oxydation des fibres kératiniques **caractérisé en ce qu'**on applique sur les fibres une composition telle que définie à l'une quelconque des revendications 17 à 22, en présence d'un agent oxydant pendant un temps suffisant pour révéler la couleur souhaitée.

24. Procédé selon la revendication 23, dans lequel l'agent oxydant est choisi parmi le peroxyde d'hydrogène, le peroxyde d'urée, les bromates de métaux alcalins, les persels, les peracides et les enzymes oxydases.

25. Procédé selon la revendication 24 dans lequel l'agent oxydant est mélangé au moment de l'emploi à la composition telle que définie selon l'une quelconque des revendications 17 à 22.

26. Procédé selon la revendication 25 dans lequel l'agent oxydant est appliqué sur les fibres simultanément ou séquentiellement à la composition telle que définie selon l'une quelconque des revendications 17 à 22 sous forme d'une composition oxydante.

27. Dispositif à plusieurs compartiments dans lequel un premier compartiment tinctoriale contient une composition telle que définie à l'une quelconque des revendications 17 à 22 et un deuxième compartiment contient un agent oxydant.

28. Utilisation d'au moins un composé du type imidazole tel que défini selon l'une quelconque des revendications 1 à 16 pour la teinture par oxydation des fibres kératiniques.
